Europäisches Patentamt

European Patent Office

Office européen des brevets

(19)

(11) Numéro de publication : **0 381 584 B1**

(12)

## FASCICULE DE BREVET EUROPEEN

(45) Date de publication du fascicule du brevet :
**12.01.94 Bulletin 94/02**

(21) Numéro de dépôt : **90400266.4**

(22) Date de dépôt : **01.02.90**

(51) Int. Cl.⁵ : **A61K 37/66**

(54) **Utilisation de certains interférons gamma pour le traitement du cancer de l'ovaire.**

(30) Priorité : **02.02.89 FR 8901346**

(43) Date de publication de la demande :
**08.08.90 Bulletin 90/32**

(45) Mention de la délivrance du brevet :
**12.01.94 Bulletin 94/02**

(84) Etats contractants désignés :
**AT BE CH DE DK FR GB IT LI LU NL SE**

(56) Documents cités :
**WO-A-87/05518
JOURNAL OF CLINICAL ONCOLOGY, vol. 6,
no. 4, avril 1988, American Society of Clinical
Oncology; R. D'ACQUISTO et al., pp. 689-695
AM. J. CLIN. ONCOLOGY, vol. 11, no. 4, 1988;
C.E. WELANDER et al., pp. 465-469**

(56) Documents cités :
**DIALOG INFORMATION SERVICES, File 155;
K. ADACHI et al., acc. no. 05608310
GEBURTSH. U. FRAUENHEILKUNDE, vol. 49,
novembre 1989, Georg Thieme Verlag, Stuttgart (DE); C. MARTH et al., pp. 987-991
INVESTIGATIONAL NEW DRUGS, vol. 7, no. 4,
1989; W. MIKSCHE et al., p. 412, no. 256**

(73) Titulaire : **ROUSSEL-UCLAF
35, Boulevard des Invalides
F-75007 Paris (FR)**

(72) Inventeur : **Brandely, Maud
135, Boulevard Malesherbes
F-75017 Paris (FR)**
Inventeur : **Lando, Danielle
17-19, rue de la Plaine
F-75020 Paris (FR)**

(74) Mandataire : **Fritel, Hubert et al
111, route de Noisy B.P. no 9
F-93230 Romainville (FR)**

## Description

Utilisation de certains interférons gamma pour la préparation de compositions pharmaceutiques destinées au traitement du cancer de l'ovaire par voie intrapéritonéale.

L'interféron gamma, outre ses propriétés antivirale et antiproliférative, possède une puissante activité immunomodulatrice qui le distingue des interférons alpha et béta. Il stimule les cellules phagocytaires permettant en particulier la lyse de certaines cellules tumorales. L'étude de la tolérance de l'interféron gamma chez des patients cancéreux au stade terminal n'a pas conduit à des observations de rémissions de ces cancers (Vadhan-Raj, S et al. (1986) J. Clin. Oncol. 4 (2) 137-146 ou Van Der Burg, M et al. (1985) J. Biol. Resp. Mod. 4 (264-272).

L'efficacité de l'interféron gamma sur diverses cellules cancéreuses fraîches humaines, selon le test dit "human tumor cloning system" décrit par Hamburger et al., a été montrée dans la demande de brevet WO 87/05518, notamment sur des colonies de cancer de l'ovaire. A la suite de ces observations, des études cliniques ont été conduites, en particulier chez des malades ayant un cancer de l'ovaire. Cependant l'efficacité de l'interféron gamma in vivo n'a pas été observée soit en administration par voie intraveineuse (Welander, C E et al. Am. J Clin. Oncol (1988) 11 (4) 465-469), soit selon un protocole utilisant l'administration par voie intrapéritonéale [(D'Acquisto, R et al. J. Clin. Oncol. (1988) 6 (689-695)]. D'une manière générale, on admet que l'action anticancéreuse de l'interféron gamma nécessite son utilisation en association avec d'autres agents thérapeutiques [(Saito, T et al. Cancer Chemother. Pharmacol. (1989) 19 (233-239)]. Or la demanderesse vient d'obtenir des résultats montrant que, dans certaines conditions d'utilisation, certains interférons gamma présentent une activité sur le cancer de l'ovaire.

L'invention concerne donc l'utilisation d'un polypeptide recombinant ayant une activité du type de l'interféron gamma humain pour préparer une composition pharmaceutique administrable par voie intrapéritonéale en perfusion et destinée au traitement efficace du cancer de l'ovaire caractérisée en ce que le polypeptide employé est un produit ayant une activité spécifique au moins égale à $1 \times 10^7$ U/mg. Ces résultats vont à l'encontre de l'absence d'efficacité rapportée par D'Acquisto, R pour un interféron gamma recombinant administré en perfusion par la voie intrapéritonéale dans le traitement de cancers ovariens réfractaires. L'invention décrit l'utilisation d'interféron gamma humain dans un traitement dont l'efficacité est montrée par un taux de réponse de 58 %, dans une administration par voie intrapéritonéale en perfusion, chez des malades atteintes de cancer de l'ovaire ayant des lésions tumorales résiduelles après chirurgie d'exérèse et chimiothérapie. L'activité spécifique des produits recombinants utilisés dans l'invention est au moins égale à $1 \times 10^7$ U/mg, déterminée selon le test classique par mesure de l'activité antivirale par comparaison avec un étalon NIH sur des cellules humaines Wish infectées par le virus de la stomatite vésiculaire et permet d'administrer des doses efficaces qui sont inférieures à la dose maximum tolérée exprimée en mg de produit. L'utilisation selon l'invention fait donc appel à des polypeptides recombinants ayant une activité du type de l'interféron gamma et possédant un haut degré de pureté. Les compositions pharmaceutiques préparées selon l'invention renferment de préférence un interféron gamma humain recombinant c'est-à-dire obtenu par la technologie de l'ADN recombinant, par exemple comme cela est décrit par Gray, et al. dans Nature (1982) 295 503-508 ou dans la demande de brevet EP 77670, des allèles ou des dérivés de ces produits tels que décrits par exemple dans la demande de brevet EP161504. On utilise ensuite des techniques de purification connues de l'homme de métier, qui permettent de préparer des produits de haute pureté. L'interféron gamma est notamment celui obtenu à partir d'une souche d'E.coli et comportant 143 aminoacides correspondant à la séquence de l'interféron gamma naturel avec une méthionine N-terminale supplémentaire.

L'invention a particulièrement pour objet l'utilisation caractérisée en ce que le polypeptide employé, de préférence l'interféron gamma, est administré à la dose de 10 à $50 \times 10^6$ U/M$^2$ par injection et plus particulièrement celle caractérisée en ce que le polypeptide employé, de préférence l'interféron gamma, est administré à la dose de $20 \times 10^6$ U/M$^2$ par injection.

L'invention a notamment pour objet l'utilisation caractérisée en ce que le polypeptide employé, de préférence l'interféron gamma, est administré de façon répétée au moins deux jours non consécutifs par semaine et tout spécialement celle caractérisée en ce que le polypeptide employé, de préférence l'interféron gamma, est administré de façon répétée pendant au moins deux mois, par exemple trois mois.

La dose administrée, la fréquence de l'injection et la durée du traitement varient en fonction de l'état du malade.

Le polypeptide employé, de préférence l'interféron gamma, est compris dans une composition pharmaceutique, de préférence lyophilisée en flacons-dose contenant de 0,2 à 1 mg de principe actif et que l'on reconstitue avec de l'eau distillée pour injection. La solution obtenue est immédiatement diluée à l'aide d'un soluté qui contribue à la stabilité du principe actif pendant la perfusion, par exemple du chlorure de sodium à 0,9 %.

A titre d'exemple, on a réalisé une préparation pour perfusion intrapéritonéale de formule :

| | |
|---|---|
| interféron gamma | 1 mg |
| excipient | 50 mg |
| eau stérile | 4,1 ml |
| chlorure de sodium à 0,9 % | 250 ml |

Selon l'utilisation préférée de l'invention, le polypeptide utilisé, de préférence l'interféron gamma, a une activité spécifique de $2 \times 10^7$ U/mg, la dose est de $20 \times 10^6$ U/M², la fréquence de l'injection est de deux fois par semaine et la durée de l'administration est de 4 mois représentant environ $720 \times 10^6$ U/M² et 36 mg/M² d'interféron gamma administrés au total par voie intrapéritonéale chez le malade.

Les résultats suivants illustrent l'invention sans toutefois la limiter :

L'étude inclue des malades ayant des lésions tumorales résiduelles de taille variant de microscopique à plus de 20 millimètres après un traitement par chimiothérapie et chirurgie d'exérése, dont l'efficacité a été évaluée à 20 % de réponse complète après un examen histologique effectué sur une deuxième opération.

Les compositions d'interféron gamma préparées selon l'invention permettent d'injecter des doses de $20 \times 10^6$ U/M², soit 1 mg/M² par injection, à raison de 2 injections par semaine pendant 2 à 4 mois, par voie intrapéritonéale selon un mode ambulatoire. On utilise les compositions décrites ci-dessus contenant 1 mg de principe actif que l'on perfuse chez le malade pendant une durée n'excédant pas 2 heures, après perfusion préalable de liquide de dialyse variant, selon l'état du malade, de 1 à 2 litres et constitué de Dianéal[R] 137 à 1,36 % de glucose en provenance de TRAVENOL.

L'évaluation des réponses des malades est faite lors d'une laparotomie par examens macroscopique et histologique avec biopsies des sites préalablement atteints et biopsies au hasard.

Sur 12 malades réévalués, les réponses suivantes ont été obtenues :

| Patient | Taille des lésions | Durée de traitement (mois) | Réponse (*) |
|---|---|---|---|
| 201 | $\geq$ 20 | 4 mois | S |
| 301 | micro | 3 mois | P |
| 503 | micro | 4 mois | RC |
| 801 | < 5 | 4 mois | P |
| 803 | < 20 | 3 mois | RP |
| 1001 | > 20 | 4 mois | S |
| 1002 | < 20 | 3 mois | RC |
| 1004 | < 5 | 2,5 mois | RC |
| 1101 | micro | 4 mois | RC |
| 1102 | micro | 4 mois | P |
| 1901 | micro | 4 mois | RP |
| 1902 | micro | 4 mois | RP |

(*) S = stabilisation,
P = progression,
RC = réponse complète,
RP = réponse partielle.

Les résultats montrent 4 réponses complètes et 3 réponses partielles soit un taux de réponse de 58 %.

**Revendications**

1. Utilisation d'un polypeptide recombinant ayant une activité du type de l'interféron gamma humain pour préparer une composition pharmaceutique administrable par voie intrapéritonéale en perfusion et destinée au traitement efficace du cancer de l'ovaire caractérisée en ce que le polypeptide employé est un produit ayant une activité spécifique au moins égale à $1 \times 10^7$ U/mg.

2. Utilisation selon la revendication 1, caractérisée en ce que le polypeptide employé est l'interféron gamma.

3. Utilisation selon la revendication 1 ou 2, caractérisée en ce que la composition pharmaceutique de l'interféron gamma est adaptée à être administrée à la dose de 10 à $50 \times 10^6$ U/M$^2$ par par injection.

4. Utilisation selon l'une quelconque des revendications 1 à 3, caractérisée en ce que la composition pharmaceutique de l'interféron gamma est adaptée à être administré à la dose de $20 \times 10^6$ U/M$^2$ par injection.


## Claims

1. Use of a recombinant polypeptide having an activity of human gamma interferon type for preparing a pharmaceutical composition which can be administered by intraperitoneal route as a perfusion and intended for the effective treatment of ovarian cancer, characterized in that the polypeptide used is a product having a specific activity at least equal to $1 \times 10^7$ U/mg.

2. Use according to claim 1, characterized in that the polypeptide used is gamma interferon.

3. Use according to claim 1 or 2, characterized in that the pharmaceutical composition of gamma interferon is adapted to be administered at a dose of 10 to $50 \times 10^6$ U/M$^2$ per injection.

4. Use according to any one of claims 1 to 3, characterized in that the pharmaceutical composition of gamma interferon is adapted to be administered at a dose of $20 \times 10^6$ U/M$^2$ per injection.


## Patentansprüche

1. Verwendung eines rekombinanten Polypeptids mit einer Aktivität vom Typ des humanen $\gamma$-Interferons für die Herstellung einer pharmazeutischen Zusammensetzung, die intraperitoneal durch Perfusion verabreichbar ist und vorgesehen ist für die wirksame Behandlung des Ovarium-Karzinoms, dadurch gekennzeichnet, daß das verwendete Peptid ein Produkt mit einer spezifischen Aktivität von zumindest entsprechend $1 \cdot 10^7$E/mg ist.

2. Verwendung gemäß Anspruch 1, dadurch gekennzeichnet, daß das verwendete Polypeptid das $\gamma$-Interferon ist.

3. Verwendung gemäß Anspruch 1 oder 2, dadurch gekennzeichnet, daß die pharmazeutische Zusammensetzung des $\gamma$-Interferons der Verabreichung in einer Dosis von 10 bis $50 \cdot 10^6$E/M$^2$ durch Injektion angepaßt ist.

4. Verwendung gemäß einem der Ansprüche 1 bis 3, dadurch gekennzeichnet, daß die pharmazeutische Zusammensetzung des $\gamma$- Interferons der Verabreichung in einer Dosis von $20 \cdot 10^6$E/M$^2$ durch Injektion angepaßt ist.